# EUROPÄISCHE PATENTSCHRIFT

(11) **EP 1 325 044 B1**
(45) Veröffentlichungstag und Bekanntmachung des Hinweises auf die Patenterteilung: **01.09.2004**
(21) Anmeldenummer: 01982350.9
(22) Anmeldetag: 25.09.2001
(51) Int. Cl.: C08F 118/08, C08F 2/24, C08F 2/38, A61K 9/28, A61K 9/50, A61K 7/42, C08F 271/02

(54) **WÄSSRIGE VINYLACETAT POLYMERDISPERSION**
AQUEOUS POLYMER DISPERSION
Dispersion aqueuse de polymere

(30) Priorität: 29.09.2000 DE 10048888; 10.05.2001 DE 10122786
(43) Veröffentlichungstag der Anmeldung: 09.07.2003
(73) Patentinhaber: BASF AKTIENGESELLSCHAFT, 67056 Ludwigshafen (DE)
(72) Erfinder: KOLTER, Karl, 67117 Limburgerhof (DE); ANGEL, Maximilian, 67105 Schifferstadt (DE); ZEITZ, Katrin, 67067 Ludwigshafen (DE)
(86) Internationale Anmeldenummer: PCT/EP2001/011053
(87) Internationale Veröffentlichungsnummer: WO 2002/026845

(56) Entgegenhaltungen:
- DE-A- 19 709 532
- FR-A- 977 296
- US-A- 5 252 704

## Beschreibung

Die Erfindung betrifft eine wässrige Polymerdispersion, hergestellt durch radikalische Polymerisation von Vinylacetat, ein Verfahren zur Herstellung einer derartigen wässrigen Polymerdispersion sowie deren Verwendung.

Aus der US 5 252 704 sind in Wasser redispergierbare Polymerpulver bekannt, die unter Verwendung von Polyvinylpyrrolidon (PVP) als Dispersionsmittel hergestellt werden. Zur Herstellung der Polymerpulver werden unter anderem auch Vinylester in üblicher Emulsionspolymerisation eingesetzt. Vor der Sprühtrocknung wird der Emulsion PVP zugesetzt. Die Polymerpulver sind vor allem als Additive für Zementmischungen vorgesehen.

In der DE 43 41 156 C1 ist die Verwendung von in Wasser redispergierbaren Kunststoffdispersionspulvern als Arzneimittelträger in Arzneiformen mit gesteuerter Wirkstoffabgabe offenbart, wobei die Pulver eine Kern-Hülle-Struktur haben mit bestimmten Tg-Werten für die Kern- bzw. Hüllpolymerisate.

In der DE 197 09 532 A ist die Verwendung von redispergierbaren Polymerpulvern oder Polymergranulaten zum Überziehen von pharmazeutischen oder agrochemischen Darreichungsformen beschrieben, wobei die Pulver bzw. Granulate aus 10 bis 95 Gew.-% Polyvinylacetat und aus 5 bis 90 Gew.-% eines N-Vinylpyrrolidon-haltigen Polymers sowie gegebenenfalls weiteren Zusatzstoffen bestehen.

Für die Herstellung von pharmazeutischen Darreichungsformen werden wie in DE 197 09 532 häufig Polymerpulver eingesetzt, die zur Herstellung dieser Darreichungsformen in Wasser redispergiert werden müssen. Die Gründe für diese Herstellung von redispergierbaren Pulvern liegen darin, dass es häufig nicht möglich ist, die wässrigen Zubereitungen entsprechend zu stabilisieren, so dass sie die hohen Anforderungen, die an Einsatzstoffe für Arzneimittel gestellt werden, erfüllen. So dürfen beispielsweise kein mikrobiologischer Befall und auch keine Teilchenvergröberung oder gar Koagulation bzw. Sedimentation auftreten, weil dadurch die sichere Herstellung des Arzneimittels gefährdet wird. Die wässrigen Zubereitungen sind oft nur über Wochen stabil. Um zu einer längeren Haltbarkeit zu kommen, werden die wässrigen Zubereitungen in Pulver überführt, aus denen wiederum vor der Anwendung durch Einrühren in Wasser eine wässrige Zubereitung herstellt werden muss. Diese Vorgehensweise kostet sehr viel Energie und Zeit und durch die thermische und mechanische Belastungen des Produktes beim Sprühtrocknen bzw. Einrühren verändern sich die ursprünglichen Eigenschaften. Außerdem ist die Reproduzierbarkeit von bedeutenden Eigenschaften der Darreichungsformen, wie z.B. der Wirkstofffreisetzung, oft schlecht, weil redispergierbare Pulver häufig schlechter haftende, nicht vollständig verfilmte, recht inhomogene Überzüge ergeben. Ein Grund hierfür ist sicherlich, dass es niemals gelingt eine 100 %ige Redispergierung der Pulver zu erhalten und solche Redispersionen deshalb immer Anteile an groben Teilchen bzw. Teilchenagglomeraten (größer als 1 µm Durchmesser) enthalten. Bereits eine Verschiebung des mittleren Teilchendurchmessers auf Werte größer 300 nm führt zu schlechten anwendungstechnischen Eigenschaften.

Feste pharmazeutische Darreichungsformen wie Tabletten, Kapseln, Pellets, Granulate, Kristalle etc. werden aus sehr unterschiedlichen Gründen gecoatet, d.h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüber hinaus lässt sich insbesondere die Freisetzungsgeschwindigkeit des Wirkstoffes durch den Filmüberzug einstellen. In ähnlicher Weise gelten diese.Kriterien auch für agrochemische Darreichungsformen.

Generell unterscheidet man Instant-Release-Formen und Retard- bzw. Slow-Release-Formen.

Bei Instant-Release-Formen soll der Wirkstoff in möglichst kurzer Zeit freigesetzt werden. Hierbei darf der Überzug die Freisetzung des Wirkstoffes aus dem Kern nicht oder nur wenig behindern.
In der pharmazeutischen Anwendungstechnik sind Instant-Release-Formen Zubereitungen, bei denen mehr als 80 % des Wirkstoffs innerhalb einer Stunde freigesetzt werden.

Bei Retard-Formen ist hingegen die Freisetzung verzögert, um beispielsweise Plasmaspiegelspitzen und damit mögliche Nebenwirkungen zu verhindern oder die Einnahmefrequenz zu verringern. Bei den sogenannten überzogenen Retard-Formen, auch Coating-Retard-Formen genannt, verlangsamt ein Filmüberzug die Freisetzung des Arzneistoffes. Hierzu werden häufig wasserunlösliche Cellulosederivate wie Ethylcellulose oder (Meth)acrylat Copolymere, insbesondere Eudragit® NE, RS oder RL (Röhm Pharma, Weiterstadt), eingesetzt. Für Eudragit® RS und RL werden Weichmacherzusätze von 10 bis 20 Gew.-%, bezogen auf den Filmbildner, empfohlen. Bei Ethylcellulose ist ein noch höherer Weichmacheranteil (ca. 30 Gew.-%) unabdingbar. Lediglich Eudragit® NE benötigt keinen Weichmacher, da es eine sehr niedrige Glasübergangstemperatur und Mindestfilmbildungstemperatur besitzt. Dadurch bedingt ist es allerdings klebrig und schwer zu verarbeiten.

Der vorliegenden Erfindung lag die Aufgabe zugrunde, Einsatzstoffe für die Herstellung insbesondere pharmazeutischer Darreichungsformen zur Verfügung zu stellen, die sehr lagerstabil sind, die sich gut verarbeiten lassen, die insbesondere gute, leicht verfilmende, homogene Überzüge mit sehr gut reproduzierbarer Freisetzung ergeben und die gegebenenfalls nicht über zunächst getrocknete und dann wieder in H₂O zu redispergierende Pulver hergestellt werden müssen.

Diese Aufgabe wird gelöst durch eine wässrige Polymerdispersion, hergestellt durch radikalische Polymerisation von Vinylacetat in Gegenwart von mindestens einem ionischen Emulgator, mindestens einem radikalischen Initiator und mindestens einem Schutzkolloid, dadurch gekennzeichnet, dass die Polymerisation in Gegenwart eines Polymerisationsreglers ausgeführt wird und dass das Gew.-Verhältnis von Schutzkolloid zu ionischem Emulgator mindestens 4 : 1, bevorzugt mindestens 8 : 1 beträgt, besonders bevorzugt im Bereich zwischen 8 : 1 und 12 : 1 liegt und dass das Gew.-Verhältnis von Vinylacetat-Monomeren zu Schutzkolloid zwischen 19 : 1 und 4 : 1, vorzugsweise zwischen 15 : 1 und 6 : 1 liegt.

Eine bevorzugte Ausführungsform der oben genannten wässrigen Polymerdispersion ist dadurch gekennzeichnet, dass sie durch eine radikalische Polymerisation bei einem pH-Wert im Bereich von 1 bis 7, besonders bevorzugt im Bereich von 3 bis 6 hergestellt wird.

Es hat sich dabei als vorteilhaft herausgestellt, wenn der pH-Wert während der Polymerisation durch Zugabe eines basisch wirkenden Reagenzes im Bereich von pH 1 bis pH 7 konstant gehalten wird. Unter einem konstant gehaltenen pH-Wert, sowohl während als auch nach der Polymerisation, ist ein pH-Wert mit Schwankungen im Bereich von +/- 1,5, bevorzugt +/- 1, besonders bevorzugt +/- 0,5 Einheiten zu verstehen.

Als basisch wirkende Reagenzien werden im Rahmen der Erfindung bevorzugt Alkalimetall- oder Erdalkalimetallhydroxide, besonders bevorzugt wässrige Lösungen eines Alkalimetall- oder Erdalkalimetallhydroxids - insbesondere Natronlauge oder Kalilauge - sowie wässrige Ammoniaklösungen verwendet.

Als besonderen Vorteil hat es sich herausgestellt, wenn der pH-Wert während der Polymerisation durch Zugabe eines Puffersystems konstant gehalten wird.

Unter Puffersysteme sind übliche Puffer und/oder polymere Puffer zu verstehen.

Geeignete Puffer sind beispielsweise alle Salze aus schwachen Säuren und starken Basen oder starken Säuren und schwachen Basen, wobei es sich um Salze derselben Säuren oder Basen oder Mischungen aus unterschiedlichen Säuren oder Basen handeln kann.

Bevorzugt wird dabei der Pufferbereich des Puffersystems zwischen pH 1 bis 7 gewählt. Als Puffer bzw. Pufferlösungen mit einem Pufferbereich im sauren Milieu zwischen pH 1 bis 7 kommen beispielsweise Puffer wie Walpole-Puffer (Essigsäure/NaAcetat, pH 3,6-5,6), Gomori-Aconitat-Puffer (Aconitinsäure/NaOH, pH 2,5-5,7), Kolthoff-Puffer (Borax/Succinat, pH 3,0-5,8), Sørensencitrat-Puffer (Dinatriumcitrat/HCl, pH 2,2-4,8) Sørensenglycin I-Puffer (Glycin, NaCl/HCl, pH 1,2-3,6), Clark und Lub-Phthalat I-Puffer (Kaliumbiphthalat/HCl, pH 2,2-3,8), Clark und Lub-Phthalat II-Puffer (Kaliumbiphthalat/NaOH, pH 4,0-6,2), Smith und Smith-Piperazin-Puffer (Piperacin, HCl/NaOH, pH 4,8-7,0), Clark und Lub-Kaliumchlorid/HCl-Puffer (KCl/HCl, pH 1,0-2,2), Gomori-Trismaleat-Puffer (Trismaleat/NaOH pH 5,2-8,6) oder Gomori-Succinat-Puffer (Succinat/NaOH, pH 3,8-6,0) in Frage. Auch Puffer wie MES, ADA, PIPES oder ACES, die in der Biochemie übliche Puffer sind, oder Aminosäurepuffer sind geeignete Puffer.

Bevorzugt werden Puffer, die sich vorteilhafterweise aus schwachen Säuren und ihren Salzen herstellen lassen, wie z.B. Natriumacetat/Essigsäure, Natriumborat/Borsäure, Natriumphosphat/Phosphorsäure, Hydrogencarbonat/Soda, Natriumhydroxid/Citronensäure, Natriumhydroxid/Weinsäure. Auch Puffer aus schwachen Basen und ihren Salzen sind geeignet. Es können einzelne Puffer oder Mischungen zur Einstellung des pH-Wertes in den Dispersionen verwendet werden.

Es ist auch möglich Puffersysteme mit einem Pufferbereich zwischen pH 7 bis 13 zu verwenden. Als Puffer bzw. Pufferlösungen mit einem Pufferbereich im basischen Milieu zwischen pH 7 bis 13 kommen beispielsweise Puffer wie Clark und Lub-Boratpuffer (Borsäure, KCl/NaOH, pH 7,8-10,0), Delory und King-Puffer (Carbonat/ Bicarbonat, pH 9,2-10,7) oder Sørensen-Glycin II-Puffer (Glycin, NaCl/HCl, pH 8,4-13) in Frage. Auch Puffer wie Cholaminchlorid, BES, TES, HEPES, Acetamidoglycin, Glycinamid, Tris, Bicine, Tricine oder Glycylglycin, die in der Biochemie übliche Puffer sind, oder Aminosäurepuffer sind geeignete Puffer.

Als Puffersystem sind auch Salze, beispielsweise Natriumsalze der Bernsteinsäure, Brenztraubensäure, Maleinsäure, Malonsäure, Äpfelsäure, Milchsäure sowie von Aminosäuren zu verwenden. Außerdem können als Puffer Salze der Polyacrylsäure, Polymethacrylsäure, Acrylsäure-Methacrylsäure-Copolymer, Carboxymethylcellulose, Carboxymethylstärke, Halbester von Cellulose, Hydrokypropylmethylcellulose oder Polyvinylalkohol mit mehrwertigen Säuren wie Phthalsäure, Bernsteinsäure oder Trimellitinsäure in Betracht gezogen werden.

Mit dieser wässrigen Polymerdispersion können, ohne dass eine Redispersion mit den damit verbundenen Nachteilen nötig ist, in einem einfachen Verfahrensschritt mit hoher Reproduzierbarkeit gut verfilmte, homogene Überzüge insbesondere auf pharmazeutische, agrochemische oder nutritive Darreichungsformen aufgebracht werden, die ausgezeichnet haften, widerstandsfähig gegen äußere Einflüsse sind und eine reproduzierbare Wirkstofffreisetzung gewährleisten. Gegenüber den in H₂O redispergierbaren Pulvern ist für die Erzielung einer bestimmten Freisetzungsrate eine niedrigere Auftragsmenge erforderlich, wodurch weitere Kosten gespart werden.

Als "nutritive Darreichungsformen" sind Tabletten, Kapseln, Granulate oder ähnliche feste Formen zu verstehen, die keine pharmazeutischen Wirkstoffe, sondern Lebensmittelergänzungsstoffe wie Vitamine, Carotinoide, Mineralstoffe, Pflanzenextrakte oder Nutraceuticals enthalten.

Die erfindungsgemäßen Zubereitungen sind unerwarteterweise unempfindlich gegenüber weiteren, üblicherweise in Sprühzubereitungen eingesetzten Hilfsstoffen wie Pigmenten, Füllstoffen, Verdickern, Suspensionsstabilisatoren, Emulgatoren, Glanzverstärker, Freisetzungsbeschleunigern etc. wie auch gegen Scherbeanspruchungen und Verfahrensschwankungen beim Coatingprozess. Aufgrund der hohen Dehnbarkeit der (Polymer-)Filme kommt es bei der Lagerung zu keinerlei Rissbildung, da die Überzüge die Formveränderungen des Kernes z.B. bedingt durch Veränderung der Umgebungsfeuchte mitmachen. Solche Überzüge sind daher auch in Gegenden mit extremer Witterung, wie Kälte oder hoher Feuchte stabil.

Überraschenderweise zeigte sich, dass die erfindungsgemäßen wässrigen Polymerdispersionen - obschon sie unter Zuhilfenahme eines Reglers hergestellt wurden und somit eher niedrige Molgewichte bzw. K-Werte aufweisen - überhaupt nicht klebrig sind und deutlich schneller auf feste Darreichungsformen aufgesprüht werden können als die bisher bekannten Zubereitungen,ohne dass es zu Agglomerationen oder Verkleben von Formlingen kommt bzw. der Überzug rauh wird. Diese erhöhte Sprühgeschwindigkeit bringt einen deutlichen Kostenvorteil in der Anwendung mit sich. Eine weitere Beschleunigung des Coatingprozesses ergibt sich durch Erhöhung der Zulufttemperatur und der Feststoffkonzentration in der Sprühlösung. Dies ist bei den herkömmlichen Zubereitungen nicht möglich.

Die mit den erfindungsgemäßen wässrigen Polymerdispersionen gecoateten Darreichungsformen weisen eine ausgezeichnete Reproduzierbarkeit in den Eigenschaften wie z.B. der Freisetzung auf, die unter anderem durch die guten Verfilmungseigenschaften bedingt ist.

Durch Zusätze von wasserlöslichen Stoffen, insbesondere von wasserlöslichen Polymeren kann die Wirkstofffreisetzung entsprechend beschleunigt werden, so dass auch schnell freisetzende Überzüge oder Überzüge zur Geschmacksmaskierung ausgebildet werden können.

Neben dem Coaten von pharmazeutischen Darreichungsformen können die erfindungsgemäßen wässrigen Polymerdispersionen, aber auch die aus ihnen in üblicher Weise hergestellten Polymerpulver, vorteilhaft eingesetzt werden, um Waschmittel- oder Geschirrspülmittelgranulate bzw. -tabletten zu beschichten.

Durch Beschichtung von oder Einarbeitung in Riech- und Aromastoffzubereitungen kann deren Freisetzung gezielt eingestellt werden und auf diese Weise die Wirkung verlängert werden.

Aufgrund der guten Sprüh- und Verfilmungseigenschaften sowie der guten Hautverträglichkeit der erfindungsgemäßen wässrigen Polymerdispersionen oder der aus ihnen hergestellten Polymerpulver eignen sich diese darüber hinaus zur Herstellung von Sprühpflastern, die mit Wirkstoffen (z.B. in Form von Desinfektions-Pumpsprays) oder ohne Wirkstoffe eingesetzt werden können, um Wunden abzudecken und zu behandeln. Hierbei ist von besonderem Vorteil, dass der Film homogen ist, die Hautatmung kaum behindert, sehr gut auf der Haut haftet, durch seine Flexibilität auch von starken Hautbewegungen nicht beeinträchtigt wird, aber auch nach Befeuchtung mit Wasser als Ganzes abgezogen werden kann, wobei auf der Haut oder Wunde keine Rückstände verbleiben. Dies ist bei den üblicherweise eingesetzten Acrylsäure-/Methacrylsäureestern nicht der Fall.

Generell eignen sich die erfindungsgemäßen wässrigen Polymerdispersionen oder die aus ihnen hergestellten Polymerpulver nicht nur für die Herstellung von Sprühpflastern, sondern für alle Produkte, die auf die Haut, Körperteile oder Gegenstände gesprüht werden sollen. Zum Beispiel können solche eingefärbten Sprühzubereitungen problemlos auf Fenster- oder Autoscheiben gesprüht werden und ergeben dann je nach verwendeter Schablone entsprechende Bilder bzw. Muster, die in einfacher Weise nach Befeuchtung mit Wasser wieder abgezogen werden können.

Die erfindungsgemäßen wässrigen Polymerdispersionen oder die aus ihnen hergestellten Polymerpulver weisen ein hohes Aufnahmevermögen für Wirkstoffe auf und können in einfacher Weise zu Transdermalen Therapeutischen Systemen verarbeitet werden, die eine gute Hautverträglichkeit besitzen. Darüber hinaus lassen sich vorteilhaft sogenannte Aknepflaster herstellen, die über Nacht appliziert, Mitesser, Pickel und Pusteln abheilen.

Die erfindungsgemäßen wässrigen Polymerdispersionen oder die aus ihnen hergestellten Polymerpulver eignen sich auch zur Herstellung von kosmetischen Zubereitungen, insbesondere Sonnenschutzzubereitungen.

Die erfindungsgemäßen wässrigen Polymerdispersionen zeichnen sich dadurch aus, dass das in ihnen enthaltene Polyvinylacetat bevorzugt einen K-Wert von 45 bis 95, insbesondere 65 bis 85 aufweist. Der jeweils gewünschte K-Wert lässt sich in an sich bekannter Weise durch die Wahl der Polymerisationsbedingungen, beispielsweise der Polymerisationsdauer und der Initiatorkonzentration in gewissem Umfang einstellen. Der K-Wert der erfindungsgemäßen Polymerisate wird durch die Verwendung eines Reglers eingestellt. Die K-Werte werden nach Fikentscher, Cellulosechemie, Bd. 13, S. 58-64 und 71-74 (1932) bei 25°C in 0,1 gew.-%iger wässriger Lösung, aber in der Praxis auch in anderen, auch nichtwässrigen Lösungen bei anderen Polymerkonzentrationen gemessen. Vorzugsweise erfolgt die Messung hier als 1 Gew.-% Polymer enthaltende Lösung in Tetrahydrofuran.

Das in der erfindungsgemäßen wässrigen Dispersion enthaltene Schutzkolloid ist bevorzugt Polyvinylpyrrolidon in einer Menge zwischen 5 und 20 Gew.-% bezogen auf Vinylacetat-Monomere, das besonders bevorzugt einen K-Wert von 20 bis 40 aufweist. Es können zusätzlich noch weitere wasserlösliche oder wasserquellbare Schutzkolloide eingesetzt werden, wie z.B. Cellulosederivate, bevorzugt Hydroxypropylmethylcellulose, Methylcellulose oder Hydroxyethylcellulose, Galactomannan, Pectin, Xanthan, Polyvinylalkohol, Acrylat-Methacrylat Copolymere, Natriumcarboxymethylstärke, Cellulose, abgebaute Stärken, Maltodextrine etc. Die Hilfsstoffe können dabei vor, während und nach der Polymerisation zugegeben werden.

Die Dispersion weist einen Feststoffgehalt von 10 bis 45 Gew.-%, bevorzugt von 15 bis 35 Gew.-% auf.

Die erfindungsgemäße wässrige Polymerdispersion ist außerdem dadurch gekennzeichnet, dass die Viskosität der Dispersion mit dem oben genannten Feststoffgehalt im Bereich von 5 bis 500 mPas, bevorzugt im Bereich von 10 bis 250 mPas, besonders bevorzugt im Bereich von 15 bis 100 mPas liegt.

Als in der erfindungsgemäßen wässrigen Dispersion enthaltener ionischer Emulgator kann ein üblicher ionischer Emulgator vorgesehen werden, wie z.B. Alkalimetall- und Ammoniumsalze von Alkylsulfaten (Alkylrest: C₈ bis C₁₆), von Alkylsulfonsäuren (Alkylrest: C₈ bis C₁₆), von Schwefelsäurehalbestern ethoxylierter Alkanole (EO-Grad: 4 bis 100, Alkylrest: C₁₂ bis C₁₆), und ethoxylierter Alkylphenole (EO-Grad 3 bis 50, Alkylrest: C₄ bis C₁₂), und von Alkylarylsulfonsäuren (Alkylrest; C₉ bis C₁₈). Als weitere anionische Emulgatoren haben sich ferner Verbindungen wie Dowfax 2A1 (Marke der Dow Chemical Company) als vorteilhaft erwiesen. Bevorzugt ist Natriumlaurylsulfat. Der ionische Emulgator wird in Konzentrationen von 0,2 bis 5 Gew.-%, bezogen auf den Gesamtmonomergehalt, eingesetzt.

Zusätzlich können noch übliche nicht-ionische Emulgatoren eingesetzt werden.

Der für die Polymerisation eingesetzte radikalische Initiator ist bevorzugt Na-, K- oder Ammoniumperoxodisulfat, aber auch andere an sich übliche radikalische Initiatoren wie Wasserstoffperoxid, organische Peroxide, Hydroperoxide oder Azoverbindungen sind - auch in Verbindung mit Redoxkomponenten wie z.B. Ascorbinsäure - möglich.

Das Gew.-Verhältnis von radikalischem Initiator zu Puffersystem beträgt bevorzugt zwischen 1 : 3 und 3 : 1.

Bei der erfindungsgemäßen wässrigen Polymerdispersion handelt es sich um eine sogenannte "geregelte" Polymerdispersion, d.h. die Dispersion wird in Gegenwart eines Polymerisationsreglers ausgeführt, wobei sich als Regler besonders schwefelhaltige Verbindungen wie z.B. Thioglykol, t-Dodecylmercaptan, n-Dodecylmercaptan und Ethylhexylthioglykolat eignen, die u.a. dazu führen, dass sich die erfindungsgemäß bevorzugten K-Werte einstellen lassen und dass die resultierenden Polymere schwefelhaltige Endgruppen aufweisen. Die Gesamtmenge des Reglers, üblicherweise zwischen 0,05 und 1 %, bevorzugt zwischen 0,1 und 0,5 %, jeweils bezogen auf den Gesamtmonomergehalt, wird bevorzugt in den Emulsionszulauf eingebracht.

Für die Herstellung von Überzügen ist - neben dem Molekulargewicht respektive dem K-Wert - die Teilchengröße der Dispersionsteilchen von besonderer Bedeutung. Die erfindungsgemäße Polymerdispersion weist daher bevorzugt Dispersionsteilchen auf, die eine mittlere Teilchengröße von nur 50 bis 300 nm, vorzugsweise von 100 bis 200 nm aufweisen. Die Bestimmung erfolgt in der üblichen Weise z.B. mittels Ultrazentrifuge, Photonenkorrelationsspektroskopie oder durch Bestimmung der Lichtdurchlässigkeit. Die Partikelgröße wird üblicherweise über die Emulgatorkonzentration gesteuert. Die erfindungsgemäß erhaltenen Dispersionsteilchen sind sehr feinteilig, obschon eine erhebliche Menge eines nichtionischen Schutzkolloids in der Vorlage der Polymerisation enthalten ist. Die US 5 252 704 lehrt dagegen, dass solche Verhältnisse bevorzugt zu grobteiligen Dispersionen führen.

Die vorliegende Erfindung betrifft auch ein Verfahren zur Herstellung einer wässrigen Polymerdispersion, bei dem Vinylacetat durch radikalische Polymerisation in Gegenwart von mindestens einem ionischen Emulgator, mindestens einem radikalischen Initiator und mindestens einem Schutzkolloid polymerisiert wird, dadurch gekennzeichnet, dass die Polymerisation in Gegenwart eines Polymerisationsreglers ausgeführt wird und dass das Gew.-Verhältnis von Schutzkolloid und ionischem Emulgator mindestens 4 : 1, bevorzugt mindestens 8 : 1 beträgt, besonders bevorzugt im Bereich zwischen 8 : 1 und 12 : 1 liegt und dass das Gew.-Verhältnis von Vinylacetat-Monomeren und Schutzkolloid zwischen 19 : 1 und 4 : 1, vorzugsweise zwischen 15 : 1 und 6 : 1 liegt. Das Gew.-Verhältnis von radikalischem Initiator zu Puffersystem liegt bevorzugt zwischen 1 : 3 und 3 : 1.

Die Polymerisation erfolgt bevorzugt bei einem pH-Wert im Bereich von 1 bis 7, besonders bevorzugt im Bereich von 3 bis 6. Je nach Ausstattung der Polymerisationsreaktoren ist es möglich, den pH-Wert während der Reaktion durch geeignete Mess- und Regelvorrichtungen auf den gewünschten pH-Wert einzustellen.

Für die,bevorzugte Ausführungsform des erfindungsgemäßen Verfahrens wird der pH-Wert während der Polymerisation durch Zugabe eines der bereits eingangs beschriebenen basisch wirkenden Reagenzien oder besonders bevorzugt durch die bereits erläuterten Puffersysteme konstant gehalten.

Es ist aber auch möglich, Vinylacetat ohne zusätzliche pH-Wert Regelung zu polymerisieren. In diesem Fall wird in der Regel nach der Polymerisation der pH-Wert der wässrigen Dispersion durch Zugabe eines der eingangs genannten Puffersysteme auf einen Wert im Bereich von 1 bis 7, bevorzugt 3 bis 6 eingestellt. Durch diese nachträgliche pH-Wert Regulierung kann die Lagerstabilität der wässrigen Polymerdispersion verbessert werden.

Die Emulsionspolymerisation wird in an sich bekannter Weise bei Temperaturen von 40°C bis 95°C drucklos oder bevorzugt bei Temperaturen von 55°C bis 80°C und einem Druck von 1,1 bis 15 bar, besonders bevorzugt bei einem Druck von 1,5 bis 6 bar durchgeführt. Der gewünschte Druck lässt sich dabei durch Einspeisung von Stickstoff vor ünd/oder während der Polymerisation, bevorzugt vor der Polymerisation in den Reaktor einstellen.

Bevorzugt wird dieses Verfahren nach einem halbkontinuierlichen Zulaufverfahren ausgeführt, wobei die Gesamtmenge des Schutzkolloids in der Vorlage vorgelegt wird. Eine weitere bevorzugte Verfahrensweise besteht darin, dass der ionische Emulgator zu mehr als 50 von 100 Teilen in der Vorlage enthalten ist.

Die vorliegende Erfindung betrifft weiterhin die Verwendung der erfindungsgemäßen wässrigen Polymerdispersionen als Hilfsstoff für pharmazeutische, agrochemische oder nutritive Darreichungsformen, insbesondere als Überzugsmittel für feste pharmazeutische, agrochemische oder nutritive Darreichungsformen. Außerdem betrifft sie die Verwendung der erfindungsgemäßen Dispersion oder der aus ihr hergestellten Pulver als Hilfsstoff, insbesondere Überzugsmittel, in Waschmitteln, Geschirrspülmitteln und Reinigungsmitteln jeglicher Art, insbesondere wenn diese als Granulate vorliegen.

Die Erfindung betrifft weiterhin die Verwendung der erfindungsgemäßen Dispersion oder der aus ihr hergestellten Pulver als Überzugs- oder Einbettungsmittel für Riech- und Aromastoffe.

Die Erfindung betrifft weiterhin die Verwendung der erfindungsgemäßen Dispersionen und der daraus hergestellten Pulver zur Herstellung von Zubereitungen, die auf Oberflächen, insbesondere die menschliche oder tierische Haut, gesprüht werden, vor allem für Sprühpflaster oder zur Herstellung von Transdermalen Therapeutischen Systemen oder auch zur Herstellung von kosmetischen Zubereitungen, insbesondere Sonnenschutzzubereitungen.

### Herstellungsbeispiel

In einem 2-1-Reaktionskessel mit Ankerrührer wurden 343,7 g Wasser, 1,8 g Na-laurylsulfat (100 %ig), 74,7 g Polyvinylpyrrolidon mit einem K-Wert von 30 (30 %ig in H₂O) und 63,5 g einer Teilmenge des Zulaufs 1 (s. untern) vorgelegt und auf 75°C aufgeheizt.

Bei 65°C wurde Zulauf 2 (s. unten) in zehn Minuten zugegeben und bei 75°C Zulauf 1 in 2 h und.Zulauf 3 (s. unten) in 3 h zudosiert.

Nach Zulaufende wurde 2 h bei 75°C nachpolymerisiert. Nach dem Abkühlen wurde mit 1 %iger NaOH auf pH ca. 5 eingestellt.

Man erhielt eine 30 %ige Dispersion (Feststoffgehalt 30 %) mit einem K-Wert von 71 (gemessen als 1 %ige Lösung in Tetrahydrofuran) und einer Teilchengröße von 121 nm. Der Koagulatanteil der Dispersion war sehr gering (0,2 g Koagulat am Rührer und im 120 µm Filter). Der pH-Wert blieb über 3 Monate unverändert.

| | | |
|---|---|---|
| Zulauf 1 | 298,8 g | VAc |
| | 1,2 g | Na-laurylsulfat 100 %ig |
| | 333,0 g | Wasser |
| | 0,9 g | Ethylhexylthioglykolat |
| | 0,75 g | Natriumacetat*3H₂O |
| | | |
| Zulauf 2 | 0,45 g | Na-peroxidisulfat |
| | 6,0 g | Wasser |
| | | |
| Zulauf 3 | 0,9 g | Na-peroxidisulfat |
| | 12,1 g | Wasser |

### Beispiel 1

### Propranolol - Retardpellets

Propranolol-HCl Pellets mit einer Korngröße 0,5 bis 1,5 mm und einem Wirkstoffgehalt von 20 % wurden in der Wirbelschicht mit der erfindungsgemäßen Polyvinylacetatdispersion (gemäß Herstellungsbeispiel) überzogen.

Die Überzugsdispersion wies folgende Zusammensetzung auf:

| | |
|---|---|
| Polyvinylacetatdispersion 30 % | 50,0 % |
| Propylenglykol | 1,7 % |
| Talkum | 5,0 % |
| Wasser | 43,3 % |

Der Feststoffgehalt der Sprühsuspension betrug 23,4 %.

Zur Herstellung der Sprühdispersion wurden Propylenglykol und Talkum in Wasser gelöst bzw. suspendiert und anschließend über eine Korundscheibenmühle homogenisiert. Diese Suspension wurde langsam in die 30 %ige Polyvinylacetatdispersion unter Rühren eingetragen. 500,0 g dieser Sprühzubereitung wurden in einem Aeromatic Strea 1 (Fa. Aeromatic) in der Wirbelschicht auf 500 g Propranololpellets aufgesprüht.

| Sprühbedingungen: | |
|---|---|
| Düse | 0,8 mm |
| Zulufttemperatur | 60°C |
| Ablufttemperatur | 35°C |
| Sprühdruck | 0,8 bar |
| Sprührate | 15 g/min |
| Trocknung | 50°C/5 min |

Der Pelletüberzug war sehr glatt und gleichmäßig. Es trat keine Zwillingsbildung auf.

Zur Bestimmung der Freisetzung wurden die gecoateten Pellets, entsprechend einer Menge von 160mg Propranalol-HCl in Gelatinekapseln gefüllt und diese über 2h in künstlichem Magensaft (0,08-N-HCl) in einem Paddle-Freisetzungsgerät (Fa. Pharmatest) bei 37°C und 50 Umdrehungen/min freigesetzt. Nach 2 h erfolgte die Umpufferung auf pH 6,8 durch Zugabe eines Phosphatpufferkonzentrates.

Folgende Freisetzungswerte wurden bestimmt:

| | |
|---|---|
| 1 h | 2 % |
| 2 h | 5 % |
| 4 h | 10 % |
| 8 h | 35 % |
| 12 h | 55 % |
| 16 h | 74 % |
| 20 h | 91 % |
| 24 h | 99 % |

Die Freisetzung der nicht gecoateten Pellets war mit 98 % nach 45 min sehr schnell.

### Beispiel 2

### Diclofenac - Retardpellets

Diclofenac-Natrium Pellets mit einer Korngröße 0,7 bis 1,5 mm und einem Wirkstoffgehalt von 30 % wurden in der Wirbelschicht mit der erfindungsgemäßen Polyvinylacetatdispersion überzogen.

Die Überzugsdispersion wies folgende Zusammensetzung auf:

| | |
|---|---|
| Polyvinylacetatdispersion 30 % | 58,0 % |
| Propylenglykol | 2,6 % |
| Wasser | 39,4 % |

Der Feststoffgehalt der Sprühsuspension betrug 20 %.

Zur Herstellung der Sprühdispersion wurde Propylenglykol in Wasser gelöst und langsam in die 30 %ige Polyvinylacetatdispersion unter Rühren eingetragen. 964,0 g dieser Sprühzubereitung wurden in einem Aeromatic Strea 1 (Fa. Aeromatic) in der Wirbelschicht auf 500 g Diclofenac-Natrium Pellets aufgesprüht.

| Sprühbedingungen: | |
|---|---|
| Düse | 0,8 mm |
| Zulufttemperatur | 55°C |
| Ablufttemperatur | 34°C |
| Sprühdruck | 1,2 bar |
| Sprührate | 18 g/min |
| Trocknung | 45°C/5 min |

Der Pelletüberzug war sehr glatt und gleichmäßig. Es trat keine Zwillingsbildung auf.

Zur Bestimmung der Freisetzung wurden die gecoateten Pellets, entsprechend einer Menge von 100mg Diclofenac-Natrium in Gelatinekapseln gefüllt und diese über 2h in künstlichem Darmsaft (Phosphatpuffer pH 6,8) in einem Paddle-Freisetzungsgerät (Fa. Pharmatest) bei 37°C und 50 Umdrehungen/min freigesetzt.

Folgende Freisetzungswerte wurden bestimmt:

| | |
|---|---|
| 1 h | 6 % |
| 2 h | 16 % |
| 4 h | 41 % |
| 8 h | 89 % |
| 12 h | 100 % |

Die Freisetzungswerte der nicht überzogenen Pellets lagen bei 99 % nach 1 h.

### Beispiel 3

### Retardierte Ascorbinsäure

Ascorbinsäure Kristalle mit einer Korngröße 0,5 bis 1,5 mm wurden in der Wirbelschicht in einem Hüttlin Kugelcoater (Fa. Hüttlin) mit der erfindungsgemäßen Polyvinylacetatdispersion überzogen.

Die Überzugsdispersion wies folgende Zusammensetzung auf:

| | |
|---|---|
| Polyvinylacetatdispersion 30 % | 50,0 % |
| Propylenglykol | 1,7 % |
| Talkum | 7,0 % |
| Sicovit Rot 30 (Eisenoxid rot) | 1,0 % |
| Wasser | 40,3 % |

Der Feststoffgehalt der Sprühsuspension betrug 26,4 %.

Zur Herstellung der Sprühdispersion wurden Propylenglykol und Talkum und Sicovit Rot 30 in Wasser gelöst bzw. suspendiert und anschließend über eine Korundscheibenmühle homogenisiert. Diese Suspension wurde langsam in die 30 %ige Polyvinylacetatdispersion unter Rühren eingetragen. 4500,0 g dieser Sprühzubereitung wurden in einem Hüttlin-Kugelcoater HKC 5 (Fa. Hüttlin) in der Wirbelschicht auf 3000 g Ascorbinsäurekristalle aufgesprüht.

| Sprühbedingungen: | |
|---|---|
| Düse | 0,8 mm |
| Zulufttemperatur | 60°C |
| Ablufttemperatur | 35°C |
| Sprühdruck | 1,2 bar |
| Sprührate | 69 g/min |
| Trocknung | 50°C/5 min |

Der Überzug war sehr glatt und gleichmäßig. Es trat keine Zwillingsbildung auf.

Zur Bestimmung der Freisetzung wurden die gecoateten Kristalle, entsprechend einer Menge von 500 mg Ascorbinsäure in Gelatinekapseln gefüllt und diese in künstlichem Magensaft(0,1-N-HCl) in einem Paddle-Freisetzungsgerät (Fa. Pharmatest) bei 37°C und 50 Umdrehungen/min freigesetzt.

Folgende Freisetzungswerte wurden bestimmt:

| | |
|---|---|
| 1 h | 4 % |
| 2 h | 9 % |
| 4 h | 28 % |
| 8 h | 49 % |
| 12 h | 69 % |
| 16 h | 85 % |
| 20 h | 98 % |

Hingegen war die Freisetzung der nicht gecoateten Kristalle sehr schnell (100 % nach 1 h).

### Beispiel 4

### Sprühpflaster

Zur Herstellung eines Treibgasaerosols, das auf der Haut bzw. auf Wunden einen Film ausbildet, werden 133,3 g 30 %ige erfindungsgemäße Polyvinylacetatdispersion in 366,7 g Ethanol eingerührt. Jeweils 50,0 g dieser Mischung werden in eine 6-OZ-Aerosoldose gefüllt und mit einem geeigneten Ventil verschlossen. Anschließend werden 50,0 g Dimethylether unter Druck in die Aerosoldose gepresst.

Nach Aufsprühen auf die Haut bildet sich ein homogener Film aus, der sehr stark haftet und sehr elastisch ist.

### Beispiel 5

### Filmbildendes Desinfektions-Pumpspray mit Cetylpyridiniumchlorid

400,0 g der 30 %igen Polyvinylactatdispersion werden mit 50,0 g einer 30 %igen Lösung von Polyvinylpyrrolidon K 30 in Wasser gemischt und gefriergetrocknet. 30,0 g dieses Pulvers werden in 570,0 g einer Ethanol/Wasser-Mischung (19:1) gelöst und unter Rühren mit 1,0 g Cetylpyridiniumchlorid sowie anschließend 399,0 g Ethylacetat versetzt. Diese Zubereitung wird in 100 ml Pumpsprayflaschen mit geeignetem Sprühkopf mit einem Hub von 0,1 ml gefüllt.

Nach dem Aufsprühen auf die Haut bildet sich ein flexibler, sehr gut haftender Film aus.

### Beispiel 6

### Aknepflaster

120,0 g 30 %ige Polyvinylacetatdispersion werden unter Rühren mit 32,0 g einer 25 %igen wässrigen Lösung von Polyvinylpyrrolidon mit K-Wert 90 versetzt. Anschließend werden 0,04 g Thimerosal und 0,5 g Dexpanthenol in 2,0 g Wasser und 8,0 g Propylenglykol gelöst und langsam unter Rühren zur Mischung aus Polyvinylacetatdispersion und Polyvinylpyrrolidon gegeben. Diese Zubereitung wird mittels eines Erichsen-Filmziehgerätes unter Verwendung eines 200-µm-Rakels auf eine 40 µm starke Polyesterfolie (Hostaphan, Fa. Hoechst) ausgerakelt. Nach Abtrocknung bei 55°C wird der Rakelprozess 2 mal wiederholt, um auf eine Schichtdicke von ca. 200 µm zu kommen. Der getrocknete Film wird mit einem silikonisierten Release Liner abgedeckt. Die Herstellung der einzelnen Pflaster mit einer Fläche von 1 cm² erfolgt durch Stanzen.

### Beispiel 7

### Transdermales Therapeutisches System mit Propranolol

40,0 g N-Pyrrolidon, 20,0 g Propranolol-HCl und 20,0 g Polyvinylpyrrolidon mit K-Wert 90 werden in 40,0 g demineralisiertem Wasser gelöst. Diese Lösung wird in 333,3 g der erfindungsgemäßen 30 %igen Polyvinylacetatdispersion unter Rühren eingearbeitet. Mit einem 200-µm-Rakel wird diese Mischung auf eine 40 µm starke Polyesterfolie ausgestrichen und bei 60°C getrocknet. Anschließend wird der Streichprozess 'zur Erhöhung der Schichtdicke nochmals wiederholt. Nach Abdeckung der Polymerschicht mit einem silikonisierten Release Liner können beliebige Formen ausgestanzt werden.

### Beispiel 8

### Sonnenschutzzubereitung

25,0 g Uvinul MC 80 (p-Methoxyzimtsäure-2-ethylhexylester), 20,0 g Tocopherolacetat, 25,0 g Isopropylmyristat und 15,0 g Cremophor RH 40 werden bei 40°C in 150,0 g Ethanol unter Rühren gelöst. Anschließend werden 50,0 g demineralisiertes Wasser, 150,0 g Propylenglykol, 50,0 g Glycerin und und 25,0 g getrocknete Polyvinylacetatdispersion unter Rühren eingearbeitet. Die Zubereitung wird auf Raumtemperatur abgekühlt und in Lotionflaschen oder Sprühflaschen abgefüllt.

## Patentansprüche

1. Wässrige Polymerdispersion, hergestellt durch radikalische Polymerisation von Vinylacetat in Gegenwart von mindestens einem ionischen Emulgator, mindestens einem radikalischen Initiator und mindestens einem Schutzkolloid, **dadurch gekennzeichnet, dass** die Polymerisation in Gegenwart eines Polymerisationsreglers ausgeführt wird und dass das Gew.-Verhältnis von Schutzkolloid zu ionischem Emulgator mindestens 4 : 1 beträgt und dass das Gew.-Verhältnis von Vinylacetat-Monomeren zu Schutzkolloid zwischen 19 : 1 und 4 : 1 liegt.

2. Wässrige Polymerdispersion nach Anspruch 1, **dadurch gekennzeichnet, dass** die Polymerisation bei einem pH-Wert im Bereich von 1 bis 7 ausgeführt wird.

3. Wässrige Polymerdispersion nach einem der Ansprüche 1 oder 2, **dadurch gekennzeichnet, dass** der pH-Wert während der Polymerisation durch Zugabe eines basisch wirkenden Reagenzes konstant gehalten wird.

4. Wässrige Polymerdispersion nach Anspruch 3, **dadurch gekennzeichnet, dass** man als basisch wirkendes Reagenz ein Alkalimetall- oder Erdalkalimetallhydroxid verwendet.

5. Wässrige Polymerdispersion nach einem der Ansprüche 1 oder 2, **dadurch gekennzeichnet, dass** der pH-Wert während der Polymerisation durch Zugabe eines Puffersystems konstant gehalten wird.

6. Wässrige Polymerdispersion nach Anspruch 5, **dadurch gekennzeichnet, dass** man als Puffersystem Salze aus schwachen Säuren und starken Basen oder Salze aus starken Säuren und schwachen Basen oder Mischungen davon verwendet.

7. Wässrige Polymerdispersion nach Anspruch 6, **dadurch gekennzeichnet, dass** es sich bei dem Puffersystem um ein Salz einer Säure, ausgewählt aus der Gruppe, bestehend aus Kohlensäure, Borsäure, Essigsäure, Zitronensäure, Weinsäure und Phosphorsäure handelt.

8. Wässrige Polymerdispersion nach einem der Ansprüche 5 bis 7, **dadurch gekennzeichnet, dass** das Gew.-Verhältnis von radikalischem Initiator zu Puffersystem zwischen 1 : 3 und 3 : 1 liegt.

9. Wässrige Polymerdispersion nach einem der Ansprüche 1 bis 8, **dadurch gekennzeichnet, dass** die Polymerisation in Gegenwart eines radikalischen Initiators, ausgewählt aus der Gruppe, bestehend aus Natrium-, Kalium- oder Ammoniumperoxodisulfat ausgeführt wird.

10. Wässrige Polymerdispersion nach einem der Ansprüche 1 bis 9, **dadurch gekennzeichnet, dass** der K-Wert des Polyvinylacetats 45 bis 95 beträgt.

11. Wässrige Polymerdispersion nach einem der Ansprüche 1 bis 10, **dadurch gekennzeichnet, dass** es sich bei dem Schutzkolloid um Polyvinylpyrrolidon handelt.

12. Wässrige Polymerdispersion nach Anspruch 11, **dadurch gekennzeichnet, dass** es sich bei dem Schutzkolloid um Polyvinylpyrrolidon mit einem K-Wert von 20 bis 40 handelt.

13. Wässrige Polymerdispersion nach einem der Ansprüche 1 bis 12, **dadurch gekennzeichnet, dass** es sich bei dem ionischen Emulgator um Natriumlaurylsulfat handelt.

14. Wässrige Polymerdispersion nach einem der Ansprüche 1 bis 13, **dadurch gekennzeichnet, dass** es sich bei dem Polymerisationsregler um eine schwefelhaltige Verbindung handelt.

15. Wässrige Polymerdispersion nach einem der Ansprüche 1 bis 14, **dadurch gekennzeichnet, dass** die mittlere Teilchengröße der Dispersionsteilchen im Bereich von 50 bis 300 nm liegt.

16. Wässrige Polymerdispersion nach einem der Ansprüche 1 bis 15, **dadurch gekennzeichnet, dass** sie einen Feststoffgehalt von 10 bis 45 Gew.-% aufweist.

17. Wässrige Polymerdispersion nach Anspruch 16, **dadurch gekennzeichnet, dass** die Viskosität der Dispersion im Bereich von 5 bis 500 mPas liegt.

18. Verfahren zur Herstellung einer wässrigen Polymerdispersion, bei dem Vinylacetat durch radikalische Polymerisation in Gegenwart von mindestens einem ionischen Emulgator, mindestens einem radikalischen Initiator und mindestens einem Schutzkolloid polymerisiert wird, **dadurch gekennzeichnet, dass** die Polymerisation in Gegenwart eines Polymerisationsreglers ausgeführt wird und dass das Gew.-Verhältnis von Schutzkolloid und ionischem Emulgator mindestens 4 : 1 beträgt und das Gew.-Verhältnis von Vinylacetat-Monomeren zu Schutzkolloid zwischen 19 : 1 und 4 : 1 liegt.

19. Verfahren nach Anspruch 18, **dadurch gekennzeichnet, dass** die Polymerisation bei einem pH-Wert im Bereich von 1 bis 7 durchgeführt wird.

20. Verfahren nach einem der Ansprüche 18 oder 19, **dadurch gekennzeichnet, dass** der pH-Wert während der Polymerisation durch Zugabe eines basisch wirkenden Reagenzes konstant gehalten wird.

21. Verfahren nach Anspruch 20, **dadurch gekennzeichnet, dass** man als basisch wirkendes Reagenz ein Alkalimetall- oder Erdalkalimetallhydroxid verwendet.

22. Verfahren nach einem der Ansprüche 18 oder 19, **dadurch gekennzeichnet, dass** der pH-Wert während der Polymerisation durch Zugabe eines Puffersystems konstant gehalten wird.

23. Verfahren nach Anspruch 22, **dadurch gekennzeichnet, dass** das Gew.-Verhältnis zwischen radikalischen Initiator und Puffersystem zwischen 1 : 3 und 3 : 1 liegt.

24. Verfahren nach einem der Ansprüche 18 bis 23, **dadurch gekennzeichnet, dass** die Polymerdispersion nach einem halbkontinuierlichen Zulaufverfahren hergestellt wird, wobei die Gesamtmenge des Schutzkolloids in der Vorlage vorgelegt wird,

25. Verfahren nach einem der Ansprüche 18 bis 24, **dadurch gekennzeichnet, dass** der ionische Emulgator zu mehr als 50 von 100 Teilen in der Vorlage enthalten ist.

26. Verfahren nach einem der Ansprüche 18 bis 25, **dadurch gekennzeichnet, dass** die Polymerisation bei einer Temperatur unter 80°C durchgeführt wird.

27. Verwendung der wäßrigen Polymerdispersion, definiert gemäß Anspruch 1, als Hilfsstoff für pharmazeutische Darreichungsformen.

28. Verwendung der wäßrigen Polymerdispersion, definiert gemäß Anspruch 1, als Überzugsmittel für feste pharmazeutische, agrochemische oder nutritive Darreichungsformen.

29. Verwendung der wäßrigen Polymerdispersion, definiert gemäß Anspruch 1 oder der aus ihr hergestellten Pulver als Hilfsstoff, insbesondere Überzugsmittel, für Waschmittel, Geschirrspülmittel und Reinigungsmittel jeglicher Art.

30. Verwendung nach Anspruch 29, **dadurch gekennzeichnet, dass** die Waschmittel, Geschirrspülmittel oder Reinigungsmittel als Granulate vorliegen.

31. Verwendung der wäßrigen Polymerdispersion, definiert gemäß Anspruch 1 oder der aus ihr hergestellten Pulver als Über-. zugs- oder Einbettungsmittel für Riech- und Aromastoffe.

32. Verwendung der wäßrigen Polymerdispersion, definiert gemäß Anspruch 1 oder der aus ihr hergestellten Pulver zur Herstellung von Zubereitungen, die auf Oberflächen gesprüht werden.

33. Verwendung nach Anspruch 32, **dadurch gekennzeichnet, dass** es sich bei der Oberfläche um die menschliche oder tierische Haut handelt.

34. Verwendung nach Anspruch 32, **dadurch gekennzeichnet, dass** die Zubereitung ein Sprühpflaster ist.

35. Verwendung der wäßrigen Polymerdispersion, definiert gemäß Anspruch 1 oder der aus ihr hergestellten Pulver zur Herstellung von Transdermalen Therapeutischen Systemen.

36. Verwendung der wäßrigen Polymerdispersion, definiert gemäß Anspruch 1 oder der aus ihr hergestellten Pulver zur Herstellung von kosmetischen Zubereitungen, insbesondere Sonnenschutzzubereitungen.

37. Verwendung nach Anspruch 36 zur Herstellung von Sonnenschutzzubereitungen.

## Claims

1. An aqueous polymer dispersion prepared by free-radical polymerization of vinyl acetate in the presence of at least one ionic emulsifier, at least one free-radical initiator and at least one protective colloid, wherein the polymerization is carried out in the presence of a polymerization regulator and wherein the ratio by weight of protective colloid to ionic emulsifier is at least 4:1 and wherein the ratio by weight of vinyl acetate monomer to protective colloid is between 19:1 and 4:1.

2. An aqueous polymer dispersion as claimed in claim 1, wherein the polymerization is carried out at a pH in the range from 1 to 7.

3. An aqueous polymer dispersion as claimed in either of claims 1 or 2, wherein the pH during the polymerization is kept constant by addition of a reagent with a basic action.

4. An aqueous polymer dispersion as claimed in claim 3, wherein an alkali metal or alkaline earth metal hydroxide is used as reagent with a basic reaction.

5. An aqueous polymer dispersion as claimed in either of claims 1 or 2, wherein the pH is kept constant during the polymerization by addition of a buffer system.

6. An aqueous polymer dispersion as claimed in claim 5, wherein salts of weak acids and strong bases or salts of strong acids and weak bases or mixtures thereof are used as buffer system.

7. An aqueous polymer dispersion as claimed in claim 6, wherein the buffer system is a salt of an acid selected from the group consisting of carbonic acid, boric acid, acetic acid, citric acid, tartaric acid and phosphoric acid.

8. An aqueous polymer dispersion as claimed in any of claims 5 to 7, wherein the ratio by weight of free-radical initiator to buffer system is between 1:3 and 3:1.

9. An aqueous polymer dispersion as claimed in any of claims 1 to 8, wherein the polymerization is carried out in the presence of a free-radical initiator selected from the group consisting of sodium, potassium or ammonium peroxodisulfate.

10. An aqueous polymer dispersion as claimed in any of claims 1 to 9, wherein the K value of the polyvinyl acetate is from 45 to 95.

11. An aqueous polymer dispersion as claimed in any of claims 1 to 10, wherein the protective colloid is polyvinylpyrrolidone.

12. An aqueous polymer dispersion as claimed in claim 11, wherein the protective colloid is polyvinylpyrrolidone with a K value of from 20 to 40.

13. An aqueous polymer dispersion as claimed in any of claims 1 to 12, wherein the ionic emulsifier is sodium lauryl sulfate.

14. An aqueous polymer dispersion as claimed in any of claims 1 to 13, wherein the polymerization regulator is a sulfur-containing compound.

15. An aqueous polymer dispersion as claimed in any of claims 1 to 14, wherein the average particle size of the dispersion particles is in the region of 50 to 300 nm

16. An aqueous polymer dispersion as claimed in any of claims 1 to 15, which has a solids content of from 10 to 45% by weight.

17. An aqueous polymer dispersion as claimed in claim 16, wherein the viscosity of the dispersion is in the range from 5 to 500 mPas.

18. A process for preparing an aqueous polymer dispersion, in which vinyl acetate is polymerized by free-radical polymerization in the presence of at least one ionic emulsifier, at least one free-radical initiator and at least one protective colloid, wherein the polymerization is carried out in the presence of a polymerization regulator and wherein the ratio by weight of protective colloid to ionic emulsifier is at least 4:1 and wherein the ratio by weight of vinyl acetate monomer to protective colloid is between 19:1 and 4:1.

19. A process as claimed in claim 18, wherein the polymerization is carried out at a pH in the range from 1 to 7.

20. A process as claimed in either of claims 18 or 19, wherein the pH during the polymerization is kept constant by addition of a reagent with a basic action.

21. A process as claimed in claim 20, wherein an alkali metal or alkaline earth metal hydroxide is used as reagent with a basic reaction.

22. A process as claimed in either of claims 18 or 19, wherein the pH is kept constant during the polymerization by addition of a buffer system.

23. A process as claimed in claim 22, wherein the ratio by weight between free-radical initiator and buffer system is between 1:3 and 3:1.

24. A process as claimed in any of claims 18 to 23, wherein the polymer dispersion is prepared in a semicontinuous feed process with the total amount of the protective colloid being present in the initial charge.

25. A process as claimed in any of claims 18 to 24, wherein more than 50 of 100 parts of the ionic emulsifier are present in the initial charge.

26. A process as claimed in any of claims 18 to 25, wherein the polymerization is carried out at a temperature below 80°C.

27. The use of the aqueous polymer dispersion as defined in claim 1 as auxiliary for pharmaceutical dosage forms.

28. The use of the aqueous polymer dispersion as defined in claim 1 as coating agent for solid pharmaceutical, agrochemical or nutritional dosage forms.

29. The use of the aqueous polymer dispersion as defined in claim 1 or of the powders prepared therefrom as auxiliary, in particular coating agent, for detergent, dishwashing and cleaning compositions of any type.

30. The use as claimed in claim 29, wherein the detergent, dishwashing or cleaning compositions are in the form of granules.

31. The use of the aqueous polymer dispersion as defined in claim 1 or of the powders prepared therefrom as coating or embedding agents for fragrances and flavorings.

32. The use of the aqueous polymer dispersion as defined in claim 1 or of the powders prepared therefrom for producing preparations which are sprayed onto surfaces.

33. The use as claimed in claim 32, wherein the surface comprises the human or animal skin.

34. The use as claimed in claim 32, wherein the preparation is a wound dressing spray.

35. The use of the aqueous polymer dispersions as defined in claim 1 or of the powders prepared therefrom for producing transdermal therapeutic systems.

36. The use of the aqueous polymer dispersions as defined in claim 1 or of the powders prepared therefrom for producing cosmetic preparations, in particular sunscreen preparations.

37. The use as claimed in claim 36 for producing sunscreen preparations.

## Revendications

1. Dispersion aqueuse de polymères, préparée par polymérisation radicalaire d'acétate de vinyle en présence d'au moins un émulsifiant ionique, d'au moins un initiateur radicalaire et d'au moins un colloïde protecteur, **caractérisée par le fait que** la polymérisation est effectuée en présence d'un régulateur de polymérisation, et que le rapport en poids du colloïde protecteur à l'émulsifiant ionique est d'au moins 4 : 1 et que le rapport en poids des monomères d'acétate de vinyle au colloïde protecteur se situe entre 19 : 1 et 4 : 1.

2. Dispersion aqueuse de polymères selon la revendication 1, **caractérisée par le fait que** la polymérisation est effectuée à une valeur du pH dans l'intervalle de 1 à 7.

3. Dispersion aqueuse de polymères selon l'une des revendications 1 ou 2, **caractérisée par le fait que** la valeur du pH pendant la polymérisation est maintenue constante par addition d'un réactif à action basique.

4. Dispersion aqueuse de polymères selon la revendication 3, **caractérisée par le fait qu'**on utilise comme réactif à action basique un hydroxyde de métal alcalin ou alcalino-terreux.

5. Dispersion aqueuse de polymères selon l'une des revendications 1 ou 2, **caractérisée par le fait que** la valeur du pH pendant la polymérisation est maintenue constante par addition d'un système tampon.

6. Dispersion aqueuse de polymères selon la revendication 5, **caractérisée par le fait qu'**on utilise comme système tampon des sels d'acides faibles et de bases fortes ou des sels d'acides forts et de bases faibles, ou leurs mélanges.

7. Dispersion aqueuse de polymères selon la revendication 6, **caractérisée par le fait qu'**il s'agit en ce qui concerne le système tampon, d'un sel d'un acide choisi dans le groupe consistant en acide carbonique, acide borique, acide acétique, acide citrique, acide tartrique et acide phosphorique.

8. Dispersion aqueuse de polymères selon l'une des revendications 5 à 7, **caractérisée par le fait que** le rapport en poids de l'initiateur radicalaire au système tampon se situe entre 1 : 3 et 3 : 1.

9. Dispersion aqueuse de polymères selon l'une des revendications 1 à 8, **caractérisée par le fait que** la polymérisation est effectuée en présence d'un initiateur radicalaire choisi dans le groupe consistant en le peroxodisulfate de sodium, de potassium ou d'ammonium.

10. Dispersion aqueuse de polymères selon l'une des revendications 1 à 9, **caractérisée par le fait que** l'indice K du poly(acétate de vinyle) vaut de 45 à 95.

11. Dispersion aqueuse de polymères selon l'une des revendications 1 à 10, **caractérisée par le fait qu'**il s'agit, en ce qui concerne le colloïde protecteur, de poly(pyrrolidone de vinyle).

12. Dispersion aqueuse de polymères selon l'une des revendications 1 à 10, **caractérisée par le fait qu'**il s'agit, en ce qui concerne le colloïde protecteur, de poly(pyrrolidone de vinyle) ayant un indice K de 20 à 40.

13. Dispersion aqueuse de polymères selon l'une des revendications 1 à 12, **caractérisée par le fait qu'**il s'agit, en ce qui concerne l'émulsifiant ionique, de laurylsulfate de sodium.

14. Dispersion aqueuse de polymères selon l'une des revendications 1 à 13, **caractérisée par le fait qu'**il s'agit, en ce qui concerne le régulateur de polymérisation, d'un composé contenant du soufre.

15. Dispersion aqueuse de polymères selon l'une des revendications 1 à 14, **caractérisée par le fait que** la granulométrie moyenne des particules en dispersion se situe dans l'intervalle de 50 à 300 nm.

16. Dispersion aqueuse de polymères selon l'une des revendications 1 à 15, **caractérisée par le fait qu'**elle présente une teneur en solides de 10 à 45 % en poids.

17. Dispersion aqueuse de polymères selon la revendication 16, **caractérisée par le fait que** la viscosité de la dispersion se situe dans l'intervalle de 5 à 500 mPa.s.

18. Procédé pour la préparation d'une dispersion aqueuse de polymères, dans lequel on polymérise de l'acétate de vinyle par polymérisation radicalaire en présence d'au moins un émulsifiant ionique, d'au moins un initiateur radicalaire et d'au moins un colloïde protecteur, **caractérisé par le fait que** la polymérisation est conduite en présence d'un régulateur de polymérisation et que le rapport en poids du colloïde protecteur et de l'émulsifiant ionique vaut au moins 4 : 1 et le rapport en poids du monomère d'acétate de vinyle au colloïde protecteur se situe entre 19 : 1 et 4 : 1.

19. Procédé selon la revendication 18, **caractérisé par le fait que** la polymérisation est effectuée à une valeur de pH dans l'intervalle de 1 à 7.

20. Procédé selon l'une des revendications 18 ou 19, **caractérisé par le fait que** la valeur du pH est maintenue constante pendant la polymérisation par addition d'un réactif à action basique.

21. Procédé selon la revendication 20, **caractérisé par le fait qu'**on utilise comme réactif à action basique un hydroxyde de métal alcalin ou de métal alcalino-terreux.

22. Procédé selon l'une des revendications 18 ou 19, **caractérisé par le fait que** la valeur du pH est maintenue constante pendant la polymérisation par addition d'un système tampon.

23. Procédé selon la revendication 22, **caractérisé par le fait que** le rapport en poids entre l'initiateur radicalaire et le système tampon se situe entre 1 : 3 et 3 : 1.

24. Procédé selon l'une des revendications 18 à 23, **caractérisé par le fait que** la dispersion de polymère est préparée selon un procédé par alimentation en semi-continu, tandis que la quantité totale du colloïde protecteur est disposée dans le récipient collecteur.

25. Procédé selon l'une des revendications 18 à 24, **caractérisé par le fait que** l'émulsifiant ionique est contenu à raison de plus de 50 pour 100 parties dans le récipient collecteur.

26. Procédé selon l'une des revendications 18 à 25, **caractérisé par le fait que** la polymérisation est conduite à une température inférieure à 80°C.

27. Utilisation de la dispersion aqueuse de polymères définie selon la revendication 1, comme adjuvant pour des formes d'administration pharmaceutiques.

28. Utilisation de la dispersion aqueuse de polymères définie selon la revendication 1, comme agent de revêtement pour des formes d'administration pharmaceutiques, agrochimiques ou nutritives solides.

29. Utilisation de la dispersion aqueuse de polymères définie selon la revendication 1 ou de la poudre préparée à partir d'elle, comme adjuvant, en particulier comme agent de revêtement, pour des lessives, des produits pour le lavage de la vaisselle et des produits de nettoyage.

30. Utilisation selon la revendication 29, **caractérisée par le fait que** la lessive, le produit de lavage de la vaisselle ou le produit de nettoyage sont présents sous forme de granulés.

31. Utilisation de la dispersion aqueuse de polymères définie selon la revendication 1 ou de la poudre préparée à partir d'elle, comme produit de revêtement ou d'enrobage pour substances odorantes et arômes.

32. Utilisation de la dispersion aqueuse de polymères définie selon la revendication 1 ou de la poudre préparée à partir d'elle, pour l'élaboration de préparations qui sont pulvérisées sur des surfaces.

33. Utilisation selon la revendication 32, **caractérisée par le fait qu'**il s'agit, en ce qui concerne la surface, de la peau humaine ou animale.

34. Utilisation selon la revendication 32, **caractérisée par le fait que** la préparation est un emplâtre par pulvérisation.

35. Utilisation de la dispersion aqueuse de polymères définie selon la revendication 1 ou de la poudre préparée à partir d'elle, pour la préparation de systèmes thérapeutiques transdermiques.

36. Utilisation de la dispersion aqueuse de polymères définie selon la revendication 1 ou de la poudre préparée à partir d'elle, pour l'élaboration de préparations cosmétiques, en particulier de préparations pour protection solaire.

37. Utilisation selon la revendication 36 pour l'élaboration de préparations de protection solaire.
